# EUROPEAN PATENT SPECIFICATION

(11) **EP 1 137 386 B1**
(45) Date of publication and mention of the grant of the patent: **03.05.2006**
(21) Application number: 99940451.0
(22) Date of filing: 23.06.1999
(51) Int. Cl.: A61K 6/00

(54) **USE OF TRICLOSAN AS AN ANTIMALARIAL AGENT**
VERWENDUNG VON TRICLOSAN ALS ANTIMALARIAMITTEL
UTILISATION DU TRICLOSAN COMME AGENT ANTI-MALARIA

(43) Date of publication of application: 04.10.2001
(73) Proprietor: Jawaharlal Nehru Centre For Advanced Scientific Research, Jakkur, Bangalore 560 064 (IN); Namita, Surolia, Bangalore 560 012 (IN); Dharmarajan, Kamalpriya, Bangalore 560 040, Karnataka (IN)
(72) Inventor: NAMITA, Surolia Indian Institute of Science, Bangalore 560 012 (IN); DHARMARAJAN, Kamalapriya, Vijaynagar 560 040, Karn (IN); NAGARAJA, Thirumalapura, Ramadhani, Bangalore 560 078, Karnataka (IN); Surolia, Avadhesha Molecular Biophysics Unit, Karnataka State (IN)
(74) Representative: Wilson, Peter
(86) International application number: PCT/IN1999/000026
(87) International publication number: WO 2001/000138

(56) References cited:
- EP-A1- 0 474 597
- DATABASE MEDLINE [Online] HEATH ET AL.: 'Mechanism of triclosan inhibition of bacterial fatty acid synthesis', XP002907177 Database accession no. 10196195 & J. BIOL. CHEM. vol. 274, no. 16, April 1999, pages 11110 - 11114

## Description

### TECHNICAL FIELD OF THE INVENTION

The present invention relates to the use of triclosan (a 2,4,4'-trichloro-2'-hydroxydiphenyl ether) for identification of a novel target as well as design of therapeutics for treatment of human *falciparum* malaria.

### BACKGROUND OF THE INVENTION

Human malaria is an extremely febrile infection caused by *Plasmodium falciparum.* An estimated 300-500 million new infections and 1.5-2.7 million deaths attributed to malaria, occur annually in the developing world. See, World Health Organization Malaria. *WHO Fact Sheet* **94,** 1-3 (1995). The disease also exacts an enormous toll in terms of lost manpower and medical expenses. Due to the widespread development of drug resistance in the parasite, insecticide-resistance in the vector, and above all non-availability of an effective malaria vaccine in near future needs identification of newer targets and development of better antimalarials.

*Plasmodium* is a parasitic protozoan belonging to phylum Apicomplexa, genus *Plasmodium,* and is transmitted by female mosquitoes belonging to the genus Anopheles. Malaria in human is caused by four species of *Plasmodium, P. falciparum, P. vivax, P. ovale and P. malariae.* The disease is characterized by periodic fevers coinciding with the liberation of merozoites during the erythrocytic phase of the infection; these fevers occur every 72 hours in the case of the *P. malariae* and every 48 hours in the other species. In all species, there is a single phase of exoerythrocytic schizogony and in *P. falciparum* and *P. malariae,* this phase lasts for 5-15 days. *Plasmodium falciparum* causes malignant tertian malaria and is the most common and serious of all forms of malaria. The infection is acute and the parasites tend to stick to endothelial cells causing blockage and cerebral damage, often resulting in death.

*Plasmodum vivax* causes benign tertian malaria and is the second most serious infection. *Plasmodium ovale* causes ovale tertian malaria and is concentrated in West Africa.

*Plasmodim malariae* causes quartan malaria and infections may last 30 years or more. Infections with these last three parasites, although debilitating, are seldom fatal in themselves. *Plasmodium falciparum,* causing cerebral malaria has become resistant to chloroquine.

The parasites of humans, with the exception of *P. malariae,* are not naturally transmissible to other animals so the malaria parasites of primates and rodents have received considerable attention both in their own rights and as models for the human infections. There are about 20 species of *Plasmodium* in non-human primates of which *P. cynomolgi,* which resembles *P. vivax,* has been the most studied. Another species from macaques, *P. knowlesi,* is now widely used in laboratory studies. The malaria parasites of rodents have been much more extensively studied than any others. These fall into two major groups, *P. berghei* and *P. yoelii* and their subspecies and *P. vinkei* and *P. chabaudi* and their subspecies. The berghei-yoelii group typically invade immature erythrocytes. They all have 24 hour periodicities and distantly have provided a wealth of information on the biology of malaria parasites which would have been otherwise unobtainable.
**Antimalarial Drugs:** The therapy for malaria includes treatment with such pharmaceuticals as quinine, chloroquine, artimisinin, mefloquine, primaquine etc. By virtue of their chemical structure and their activity on different stages of malaria life cycle, it is possible to classify currently used antimalarial drugs into three groups. These are the (a) antimetabolites (b) the 8-aminoquinolines and the (c) blood-schizontocides (d) in addition it is possible to recognize another group, antibacterial antibiotics with antimalarial activity.

### (a) Antimetabolites:

These drugs act on the folic acid cycle and are classified as type I and type II antifolates. Malaria parasites carry out pyrimidine synthesis *de novo,* in which reduced folic acid derivatives are a vital cofactor. They cannot utilize preferred pyrimidines, using salvage pathways like mammalian cells, although they possess salvage pathway for purines, hence they are sensitive to these antifolates.
**Type I antifolates** including sulphonamides and sulphones (such as sulfadoxine and dapsone) are structural analogs ofp-amino benzoic acid, H₂N 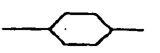 COOH Benzoic acid competes with this anti-metabolite for the active site of the enzyme dihydropteroate synthase, which joins PABA to pteridine to form dihydropteroate. A further enzyme dihydrofolate synthase links glutamate to dihydropteroate to give dihydrofolate (dihydropteroylglutamate). This pathway from PABA to dihydrofolate, is unique to microorganisms. In mammalian cells, dihydrofolate is obtained by the reduction of dietary folic acid accounting for the selective toxicity of sulphonamides and sulphones for microorganisms, and their relative safety in the mammalian host.
**Type II antifolates** (such as pyrimethamine, trimethoprim and cycloguanil the metabolite of proguanil) have similar structures to that of folate, competing with this metabolite at the enzyme dihydrofolate reductase (tetrahydrofolate dehydrogenase). The mammalian host produces tetrahydrofolate by a similar enzyme. The basis of selective toxicity of pyrimethamine and cycloguanil is the higher affinity of these compounds to plasmodial enzyme than for the mammalian one and the fact that parasite undergoes rapid proliferation as compared to cells of the host.

### b) 8-Aminoquinolines:

The 8-aminoquinolines, in particular the least toxic of this group, **PRIMAQUINE** are gametocytocides and hypnozoitocides. 8-Aminoquinolines also have activity on asexual blood stages, though with significant toxicity. It is known that 8-aminoquinolines require metabolic activation for its mode of antimalarial effect. Much work on active metabolites of primaquine is needed to pinpoint its actual mode of action, but it is suggested that treatment with 8-aminoquinolines cause swelling of mitochondria in malarial parasites. See, R.L. Beaudoin and M. Aikawa *Science* **160,** 1233-1234 (1968).

### c) Blood schizonotocides:

The blood schizonotocides, including the oldest **quinine** which is still indispensable as antimalarial are active on red cell stages of malaria parasite. These are used for active antimlarial therapy as well as in suppressive prophylaxis. The importance of hemoglobin digestion in susceptibility to these drugs is well known.

These blood schizontocides, can be divided into two groups, based on their chemical structure and their effects on the intraerythrocytic parasite.
i) The first group comprises of synthetic 4-aminoquinoline, chloroquine and the acridine mepacrine. These agents have a marked and rapid effect on the hemoglobin-containing digestive vesicles of the intraerythrocytic parasite.
ii) The second group of schizontocides are aryl amino alcohols, such as **quinine and mefloquine,** where the aryl group may or may not be a nitrogen heterocycle. They do not cause such marked or rapid effects as the first group, although they will competitively inhibit autophagic vacuole formation produced by such drugs.

### Chloroquine:

For more than 40 years, chloroquine (CQ) has been antimalarial of choice because its activity against all the four plasmodia that infect humans (*Plasmodium falciparum, P. vivax, P. ovale and P. malariae*). In addition, CQ is the only antimalarial drug known to be safe for children and pregnant women. Due to emergence of CQ-resistant strains of *P. falciparum,* the value of CQ has been severely compromised. Thus although CQ remains safe, CQ resistance has increased the morbidity and mortality from malaria. See A.A. Asindi et al., *Trop. Geogr. Med.* **45,** 110-113 (1993). This has led to the widespread use of alternative antimalarials (mefloquine, halofantrine, artemisnin derivatives and pyrimethamine-sulfadoxine (fansidar).

Although CQ has played a pre-eminent role over the years, its mode of action remains controversial. See, S.R Meshnick *An. Trop. Med Parasitol.* **90,** 367-372 (1996). Also there is disagreement about the mechanism of CQ resistance. Despite the practical limitations imposed by CQ resistance and theoretical uncertainties about the mechanisms of CQ action and resistance, there are compelling reasons to consider **aminoquinolines (AQs)** similar to CQ as potential antimalarials. If these AQs are effective in humans, they can be synthesized in developing nations by using strategies similar to the nucleophilic substitution reaction used to produce CQ. See, D. De et al., *J. Heterocycl. Chem.* **34,** 315-320 (1997). **AQs** could provide an economical alternative to the expensive antimalarial drugs now used for the treatment of CQ-resistant malaria. Although pyrimethamine-Sulfadoxine is relatively economical, its widespread use as a first line antimalarial may select rapidly for resistant parasites. See, O.C. Nwanyanwu et al., *Trop. Med Intl. Health* **21**, 231-235 (1996).

### MODE(S) OF CQ ACTION:

A number of mechanisms of action of chloroquine have been proposed which include:
i) DNA interacalation ii) elevation of vacuolar pH iii) binding of CQ to free heme iv) inhibition of aspartic and cysteine protease activity and v) Inhibition of parasite protein synthesis.

### Morphologic observations of CQ-Treated Parasites:

It is reported that CQ produces morphologic alterations in the parasite food vacuole. After exposure to CQ, the food vacuoles of CQ-susceptible malaria parasites enlarge and have reduced amounts of malaria pigment as compared to untreated controls. In addition, the parasite cytoplasm demonstrates ribosomal aggregation, mitochondrial swelling and swelling of rough endoplasmic reticulum. These effects are stage specific and are observed only with maturation of trophozoites as they interfere with normal function of food vacuole.

### Function of the food vacuole and CQ accumulation in Malaria parasites

The food vacuole of malaria parasites functions as a secondary lysosome. The red blood cell (RBC) cytoplasm, which primarily contains hemoglobin, is internalized by endocytosis and routed to the food vacuole, where hemoglobin is degraded by aspartic and cysteine proteases (plasmepsins, falcipain) to release peptides and amino acids which the parasite utilizes for protein synthesis. See, S.E. Francis et al., *Annu. Rev. Microbiol.* **51**, 97-123 (1997). This process is most active in trophozoite, when the protein synthesis is maximum. Malaria parasitized RBCs accumulate more CQ than unparasitized RBC and it is known that accumulation of CQ is essential for CQ's antiplasmodial activity. Taken together, it is suggested that CQ inhibits parasite maturation as a result of its accumulation in the food vacuole. Parasite food vacuole is a unique target as it does not exist in mammalian cells.

### Mechanisms by which CQ may interfere with the function of the food vacuole.

There are at least four mechanisms by which CQ could interfere with the function of the food vacuole.
i) **Raising vacuolar pH:** It is proposed that chloroquine, due to weak base effects and / or transport mechanisms causes alkalinization of food vacuoles. See, C.A. Homewood et al., *Nature* **235,** 50-52 (1972), D.J. Krogstad et al., *J. Cell. Biol.***101***.* 2302-2309 (1985); P.H. Schlesinger et al., *Amtimicrob. Agents Chemother.* **32,** 793-798 (1988).
ii) **Inhibiting aspartic and cysteine protease activity:** The *P. falciparum* food vacuole contains atleast three proteases for hydrolysis of host hemoglobin to heme and globin, the cysteine protease falcipain. See, P.J. Rosenthal et al., *J*. *Clin. Invest.* **82,** 1560-1566 (1988); I.Y. Gluzman et al., *J. Clin. Invest.* **93,** 1602-1608 (1994) and the aspartic proteases plasmepsin I and plasmepsin II: See, J.L. Hill et al., *FEBS Lett.* **352,** 155-158 (1994). Each of these enzymes probably participate in globin hydrolysis as the hydrolysis of hemoglobin by parasite and food vacuole extracts is inhibited in an additive manner by cysteine and aspartic protease inhibitors.
   The precise role of the three food vacuole proteases in hemoglobindegradation are unclear, due, in part, to uncertainties regarding the redox state of the food vacuole. Inhibition of both cysteine and aspartic proteases have antimalarial effects but they appear to act differently.
   Aspartic as well as cysteine protease inhibitors are under study as potential antimalarial agents. Effective antimalarial protease inhibitors should ideally inhibit parasite proteases but not host analogues such as the cysteine proteases cathepsins L and B and the aspartic protease cathepsin D.
   As both cysteine and aspartic proteases degrade hemoglobin in the food vacuole, and as inhibitors of both classes of enzymes exert antimalarial effects, and optimal chemotherapeutic strategy might involve combination therapy with inhibitors of both classes of proteases.
**iii) Binding to free heme:** It is proposed that CQ forms a toxic complex with heme and thus prevents heme from being detoxified.
**(iv) Blocking of the formation of hemozoin:** Degradation of hemoglobin: Erythrocytic malaria parasites reside in an intracellular environment that shelters them from immunologic attack but also separates them from the ready supply of nutrients circulating in the blood stream. The parasites have developed the means of acquiring nutrients from the erythrocyte cytosol.
Erythrocytic parasites take up and degrade large quantities of hemoglobin in the acid food vacuole into heme and globin. See, P.J. Rosenthal and S.R. Meshnick, *Mol. Biochem. Parasitol.* **83**, 131-139 (1996). Further hydrolysis of globin provide most of the amino acids required for parasite protein synthesis. Hemoglobin processing yields significant concentrations of free heme, which must be modified or sequestered to avoid parasite toxicity. This free heme is polymerized to hemozoin as a detoxification mechanism. The composition of hemozoin remains somewhat controversial. It appears that hemozoin consist of polymerized heme in the form of β-hematin, a noncovalent co-ordination complex with the ferric iron of each heme moiety bound to a carboxyl side chain of the adjacent heme molecule. Whether heme polymerization is catalyzed by an enzyme heme polymerase or it is a non-enzymatic reaction is another area of controversy. See, A.F.G. Slater and A. Cerami, *Nature* **355,** 167-169 (1992) and K. Raynes et al. *Biochem. Pharmacol.* **52**, 551-559 (1996).

Most of these hypotheses are unlikely to fully account for the antimalaria action of chloroquine for the following reasons.
a) The change in pH elicited by pharamacologic concentrations of chloroquine is probably too small to account fully for the action of the drug and the relative abilities of quinolines to alkalinize the food vacuole do not correlate with their antimalarial potencies.
b) There is no direct evidence in treated parasites of the quantities of free heme necessary to form toxic heme-chloroquine complexes.
c) The morphological effects of cysteine protease inhibitors are not identical to those caused by chloroquine. See, P.J. Rosenthal, *Exp. Parasitol.* **75**, 255-260 (1995).

### Alternative mechanisms by which CQ could inhibit parasite growth.

### i) Intercalation with DNA

For many years, this hypothesis was viewed as the most likely explanation for the effect of CQ on malaria parasites. The factors which argue against the hypothesis are the specificity and concentration dependence of CQ's antiparasite activity. CQ inhibits DNA synthesis at millimolar concentration in bacteria, viruses and mammalian cells. This millimolar CQ concentrations that inhibit DNA synthesis are five to six orders of magnitude (10⁵ to 10⁶) higher than the low nanomolar concentrations which inhibit parasite growth (the most striking aspect of CQ action on the parasite is its specificity at nanomolar concentrations).

### ii) Inhibition of parasite protein synthesis

It was proposed by one of the inventors (Namita Surolia) that CQ inhibits parasite protein synthesis at therapeutic concentrations by sequestering heme, which is needed for optimal protein synthesis. See, N. Surolia and G. Padmanaban, *Proc. Natl. Acad Sci. USA* **88,** 4786-4790 (1991). The deficiency of heme thus created cause enhanced autophosphorylation of eIF-2α kinase or Heme Regulated Inhibitor (HRI) which in turn inhibits protein synthesis by phosphorylating the smallest subunit of translation initiation factor, the eIF-2α. The inventor proposed that inhibition of parasite proteins synthesis is a very early event responsible for parasite death (other mechanisms are based on effects of CQ after 24 hrs of incubation or treatment with the drug, whereas inhibition of protein synthesis can be observed just after 15-20 minutes incubation of drug with the parasite). Moreover, CQ exerts its action in cytoplasm, the site of action on protein synthesis, which is, before it can reach food vacuole, the site for other proposed mechanism of action.

The inventor was also able to demonstrate the activity of enzymes viz. ALA dehydratase and ALA synthase involved in *de novo* biosynthesis of heme required for protein synthesis and as a cofactor in hemoproteins like cytochrome P-450 and cytochrome C oxidase. See, N. Surolia and G. Padmanaban, *Biochem. Biophys. Res. Commun.* **197**, 562-569 (1993).

### Mechanism of action of different pharmaceuticals.

**a. Quinine:** Quinine is a quinolinemethanol. Quinidine is the pure d isomer of quinine and is more potent. Like most other antimalarial drugs quinine is thought to affect the formation of hemozoin, the malaria pigment. The interaction of quinine with heme has been known since 1937.
**b. Artemisinin (Qinghaosu):** After quinine, artemisinin remains essential components of our antimalarial armamentarium. *Artemisia annua* (sweet wormwood) is known as qinghao to Chinese herbal medicine practitioners for at least 2,000 years. The active, water insoluble ingredient is known as Qinghaosu or artemisinin. The two artemisinin derivatives are artemether and artesunate. These Chinese drugs were not readily available for studies outside China as they were cultivated only in China, secondly many of the original Chinese studies were not acceptable to Western governments because they did not follow many of the standard practices required for clinical trials.

### Mechanism of action of Artemisinin:

There is a growing consensus that the mechanism of action involves heme and the generation of carbon-centered free radicals. It is hypothesized that artemisinin acts by generating free radicals. The generation of free radicals from artemisinin is found to be iron dependent. Alkylation of some of the parasite specific proteins by artemisinin is suggested to be the mechanism of action of artemisinin. How does protein alkylation lead to parasite death? Further elucidation of the structures and functions of the artemisinin target proteins should answer this question.

### Drugs in the pipeline:

There has been little economic incentive for private pharmaceutical firms to undertake development on antimalarial drugs. The process of antimalarial drug discovery and design is extraordinarily long and complex and our immediate concern is for the next generation of antimalarial drugs. Current drugs in the development are illustrated in the figure below:

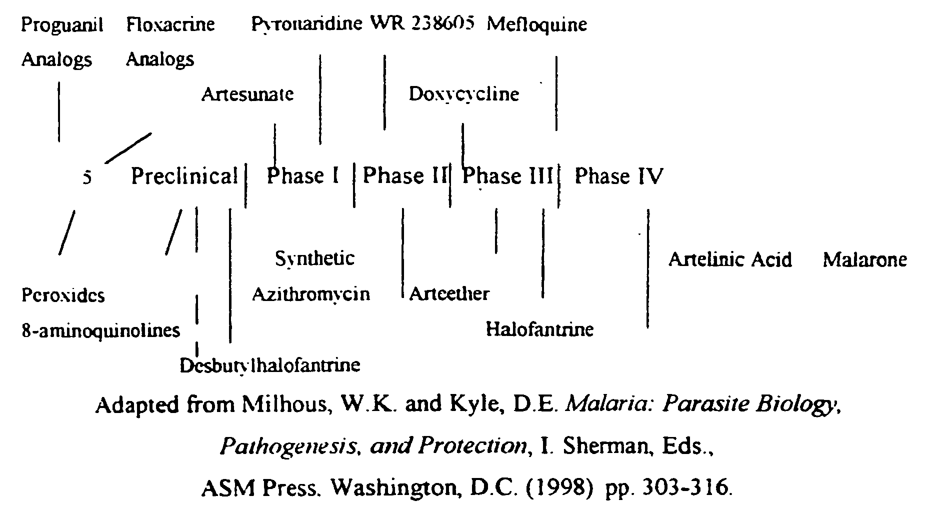

### SUMMARY OF THE INVENTION:

Triclosan, a hydroxydiphenylether, has been shown by the inventors to inhibit the growth of human malaria parasite (*P. falciparum*) *in vitro* in a potent manner. Moreover, it is also demonstrated by the investigators that the injection of triclosan in mice infected with *P. berghei* (rodent malaria parasite) is able to curtail the parasitemia and prolong their survival. More specifically, the present investigation provides evidence for (1) Triclosan abrogation of the growth of the human malaria parasite *in vitro* and the murine malaria parasite *in vivo* and identifies this compound as a prospective therapeutic (2) provides evidence that Triclosan inhibits the incorporation of [¹⁴C] or [³H] malonyl-CoA in fatty acid synthesis (3) provides evidence from the shortening of the fatty acid chains synthesized using [¹⁴C] or [³H] malonyl-CoA, a committed precursor in the fatty acid synthesis pathway thereby proving that triclosan targets enoyl-ACP reductase (FabI) in the malaria parasite.

Since, the enzymes involved in fatty acid synthesis in the malaria parasite differ from that of the human host, the present invention paves the way for developing drug that are targeted towards an essential element (FabI) of the survival mechanism of the malaria parasite.

In a first aspect the invention relates to the use of Triclosan (2, 4, 4- trichloro-2-hydroxydiphenyl ether) or a pharmaceutically acceptable derivative thereof for the manufacture of a medicament to inhibit the growth of the malaria parasite human *Plasmodium falciparum* or murine *Plasmodium berghei.*

The use may be in targeting the Fab I enzyme, enoyl-ACP reductase, in the malaria parasite. Preferably the use is in inhibiting the elongation reaction of fatty acid synthesis in malaria parasite.

The medicament is suited for treatment by introduction in the blood by any method or for application by any injectable route, preferably intramuscular or intradermal or intraperitoneal or intravenous or intro-arterial or subcutaneous.

Preferably the medicament comprises an injectable composition consisting of Triclosan (2, 4, 4- trichloro-2-hydroxydiphenyl ether) or a pharmaceutically acceptable derivative thereof and a pharmaceutically acceptable adjuvant, or a diluent or a carrier, the composition being suitable for introduction in the blood by any method or for application by any injectable route, preferably intramuscular or intradermal or intraperitoneal or intravenous or intro-arterial or subcutaneous route.

In a further aspect of the invention there is provided any method of testing that may be used to confirm that the growth of human malaria parasite Plasmodium falciparum is inhibited by the use Triclosan (2, 4, 4- trichloro-2-hydroxydiphenyl ether), comprising
a. Examining smears of *in vitro* treated cultures for morphological features of the parasite as an indicator of growth, or
b. Monitoring the incorporation of [³⁵S] methionine in infected red blood in vitro as a quantitative indicator of the inhibition of the parasite growth.

In a further aspect of the invention there is provided a method of determining the growth of animal malaria parasite murine Plasmodium berghei is inhibited by the injectable composition comprises: monitoring the extent of inhibition of parasitemia by examining the smears of blood taken from an animal.

### Brief description of Figures:

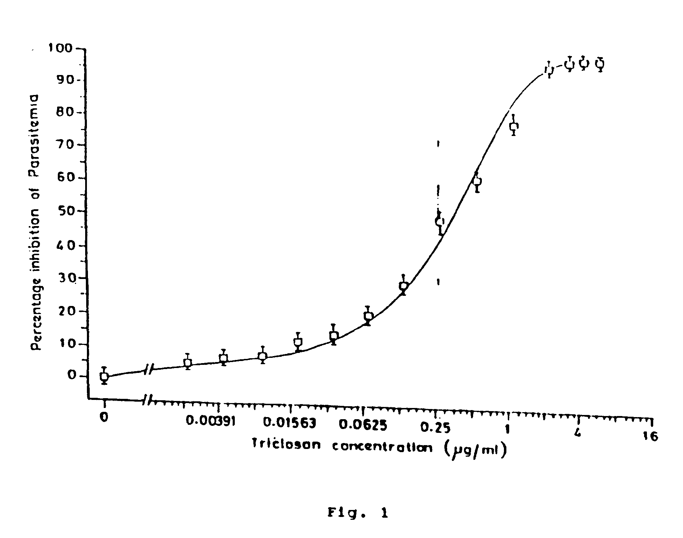

**Fig.1** Shows percentage inhibition of parasitemia after incubating *P. falciparum in vitro* with different concentrations of triclosan.

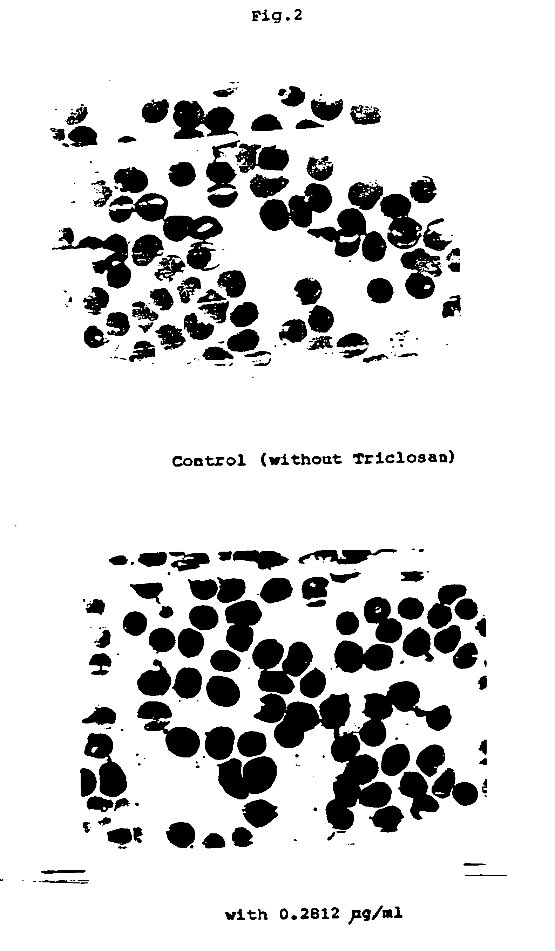

**Fig. 2** Photographs showing effect of triclosan on growth and the morphology of *P*. *falciparum* in cultures after 24h. of incubation.

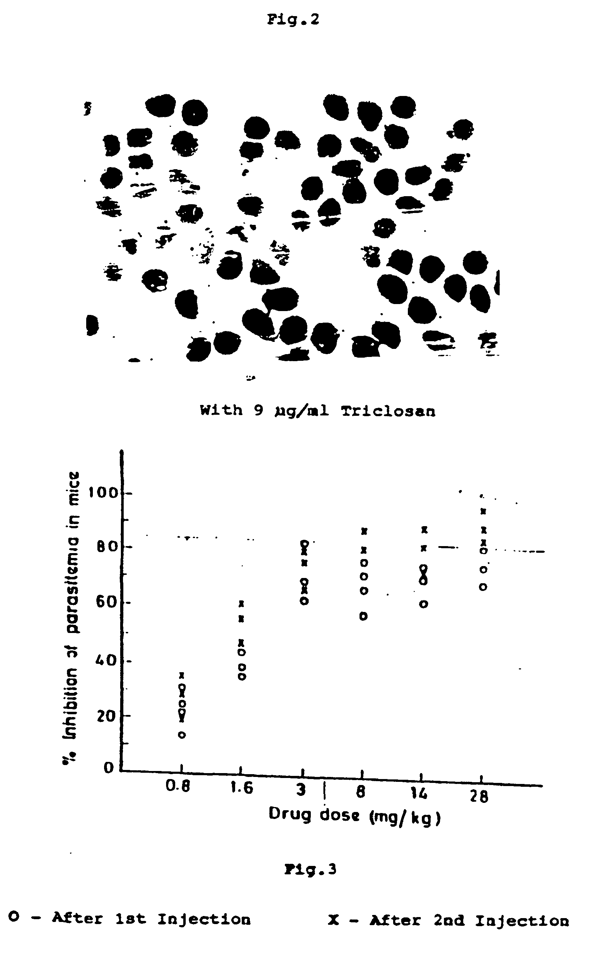

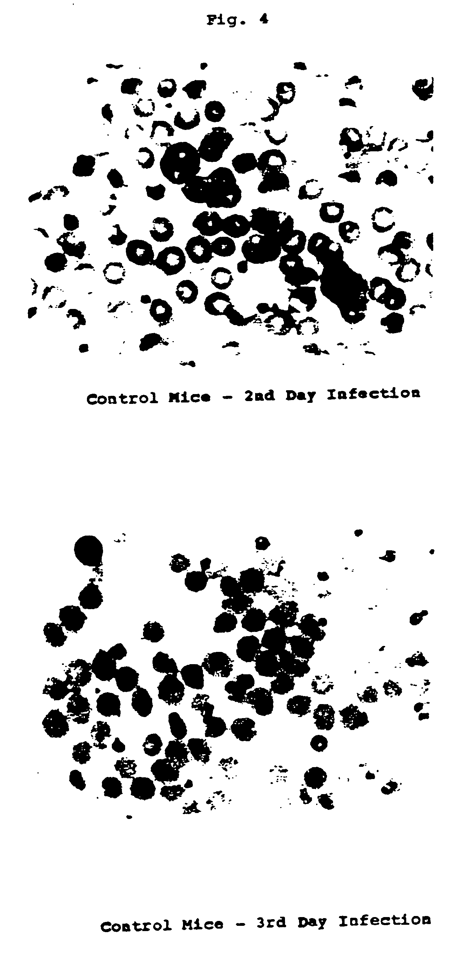

**Fig.3** Shows the efficacy of triclosan in controlling parasitemia *in vivo* in *Plasmodium berghei* infected mice, triclosan was administered subcutaneously on day one and two after the infection. The results show parasitemia after 1^{st} and 2^{nd} injections. Each point represent a separate experiment with 4-5 animals.

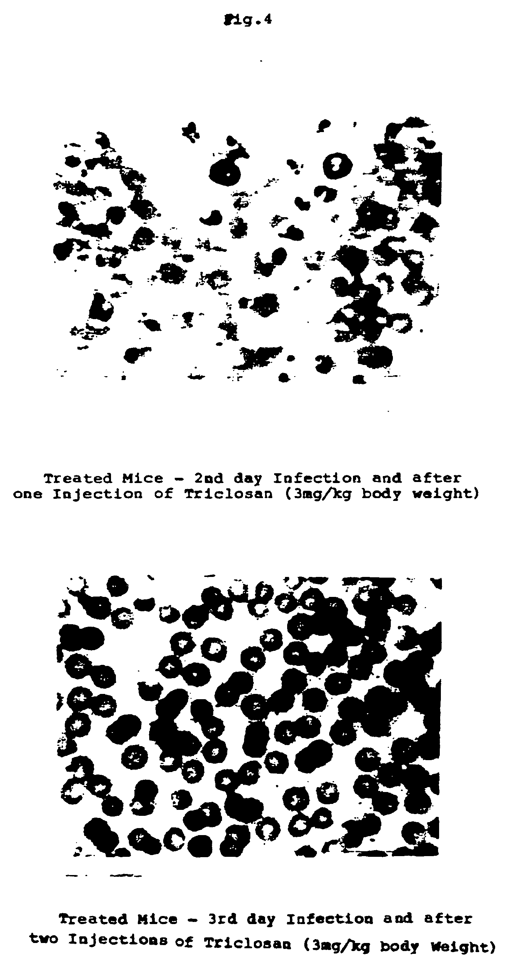

**Fig. 4** Photograph showing effect of triclosan on growth of parasites *in vivo* in *Plasmodium berghei* infected mice, after administering one and two doses of triclosan respectively (3mg/kg body weight).

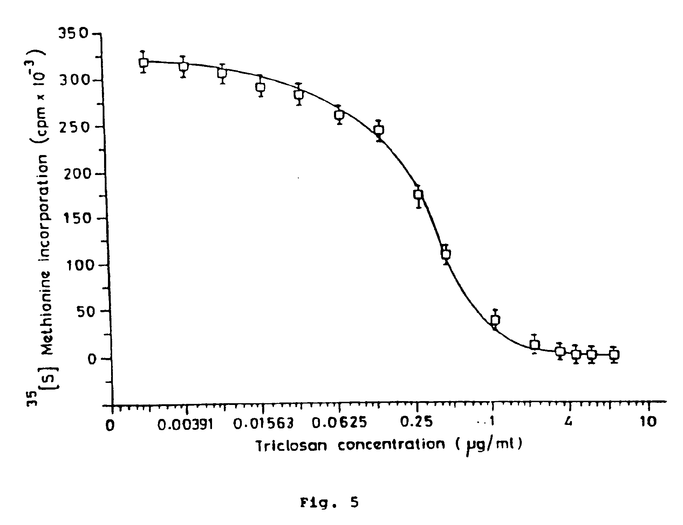

**Fig. 5** Shows inhibition of protein synthesis as demonstrated by a reduced uptake of (³⁵S) methionine by *P. falciparum* in culture with different concentrations of triclosan.

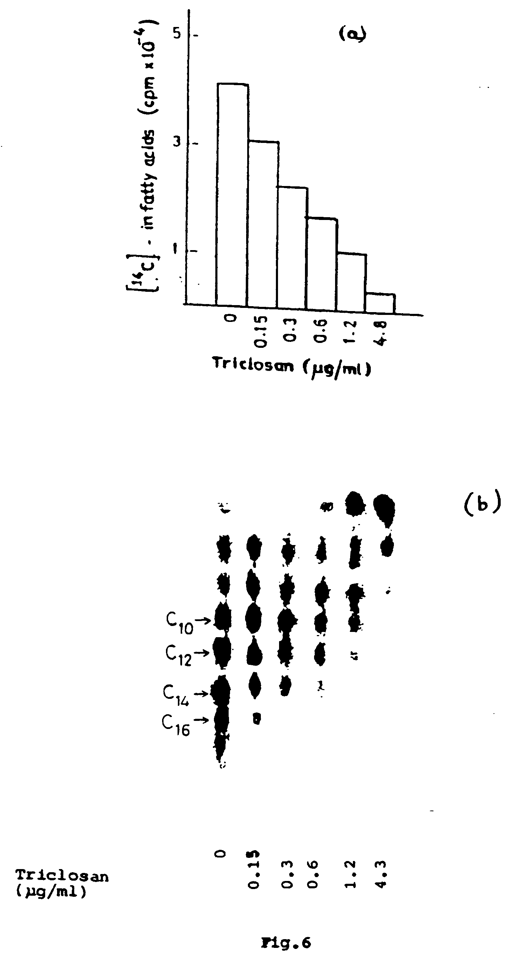

**Fig. 6a.** Effect of 0.15, 0.3, 0.6, 1.2 and 4.8 µg of triclosan on the incorporation of [¹⁴C]malonyl-CoA in fatty acids in cell free system for the synthesis of fatty acids of *Plasmodium falciparum, in vitro*.
**Fig. 6b.** Shows thin layer chromatography (TLC) results showing the effect of the above concentrations of triclosan on the type of fatty acid synthesized in *P. falciparum in vitro.*

### DESCRIPTION OF THE SPECIFIC EMBODIMENTS

### Definitions:

**Smear** refers to thin or thick film of blood on a slide. This film is air dried, fixed in methanol and is stained to examine the morphological features of parasite under the microscope.
**RPMI 1640** refers to synthetic media used in culturing parasite and other cells in tissue culture.
**CRPMI** refers to complete RPMI medium, when 25 mM Hepes buffer, 0.2% NaHCO₃ and 10% human serum is added to RPMI 1640.
**Acid Citrate Dextrose (ACD)** refers to citrate solution, an anticoagulant, used for collecting red blood cells. The composition of ACD is as follows:

| | |
|---|---|
| Tris-Sodium Citrate | 22.0 g |
| Citric acid | 8.0 g |
| Dextrose | 24.59 |

Distilled water to 1000 ml. 7.5 ml of ACD is used per 50.0 ml whole blood. This may be stored at 4°C for up to 4 weeks. Webster et al., *Application of Genetic Engineering to Research on Tropical Disease Pathogens with Special Reference to Plasmodia.* A laboratory manual of selected techniques. S. Panyim, P. Wilairat and Yuthavong, Eds. *WHO. Geneva* (1985) pp. 437.
**Parasitemia** refers to number of parasites per hundred red blood cells. Expressed as percent parasitemia after counting number of parasites in one thousand red blood cells of 10 different fields and taking mean of the values. Smears are stained with Giemsa.
**IC**_{**50**} refers to that concentration of drug which kills 50% parasite.
**Hematocrit** refers to packed red blood cell volume; and is expressed as %.
**MIC** refers to that minimum concentration (in Molar or absolute weight/ml) of drug/inhibitor/molecule which inhibits growth of parasite.
*in vitro* refers to experiments done in glass, outside a whole living organism or body; with all the components and conditions required to mimic the intact system.
*in vivo* refers to the experiments carried out in the whole body/organism.
**Autoradiography** refers to the detection of radioactive compounds by blackening of a photographic film kept in contact, under darkness, with the thin layer chromatogram of separated radioactive components, for example, the radio labeled fatty acids synthesized and separated by thin layer chromatography. See F - Turnowsky et al., *J-Bacteriol.* **171**,6555-6565 (1989).

### Different stages of Plasmodium in red blood cells:

The life cycle of malaria parasite comprises two stages 1)an asexual phase occurring in man and 2) a sexual phase occurring in the mosquito. In the asexual phase, the parasite multiplies by division, a process designated schizogony. In humans, schizogony occurs in two locations, in the red blood cells (erythrocytic schizogony) and in the liver cells (exoerythrocytic schizogony). The products of schizogony whether erythrocytic or exoerythrocytic are called merozoites.

### The human phase:

When an infected female Anopheles mosquito bites, man gets infected. The sporozoites present in the salivary gland of mosquito get injected into the human blood stream. Within an hour of being injected, the sporozoites reach the liver and enter the hepatocytes. These sporozoites undergo repeated nuclear division which is called schizont stage. In 5.5 to 15 days, the schizonts become mature and burst, releasing thousands of merozoites which enter the blood stream and infect the red blood cells. The parasite has the following stages during its growth in red blood cells.

### Ring stage:

Once inside the red cell, the merozoite rounds up and loses its internal organelles. It appears as a rounded body having vacuole in the centre, with the cytoplasm pushed to the periphery and the nucleus situated at one pole. When stained with Giemsa, the cytoplasm of ring stains blue and the nucleus red, this gives the parasite an annular or signet ring appearance. These young parasites are therefore called the ring forms.

### Trophozoite stage:

As the ring develops, it enlarges in size, becoming irregular in shape, and shows amoeboid motility. This is called the amoeboid form, which after reaching a certain stage of development, starts dividing, and is called the trophozoite. These trophozoites also contain hematin pigment-the malaria pigment or hemozoin.

### Schizont stage:

From the time the nucleus starts dividing, the parasite within the erythrocyte is called the schizont. First, only the nucleus divides into a variable number of small nuclei, the cytoplasm remaining entire and undivided. This stage is called the early schizont. When each daughter nucleus becomes surrounded by cytoplasm, it is called late schizont. The mature schizont is the fully grown form, in which 10-13 nuclei surrounded by the cytoplasm are seen. The mature schizont bursts releasing the merozoites into the circulation. The rupture of the mature schizont also releases large quantities of pyrogens, responsible for the febrile paroxysms, characterizing malaria.

### Synchronization:

*In vitro* culture of *Plasmodium falciparum* can be synchronized (adjusted to the same stage) from mixed stages, by using 5% D-sorbitol as a lytic agent for the cells containing late trophozoites and schizonts. See, C. Lambros & J. Vanderberg, *J. Parasitol* **65**, 418-420 (1979), The cells containing rings and early trophozoites (24 h after merozoite invasion) are allowed to develop synchronously.

The cultures having predominantly rings (7-8%) are centrifuged at 1300 rpm for 7 min and the supernatant is discarded. 5 volumes of 5% D-sorbitol is added to packed red cells and the mixture is incubated at room temperature for 5 min. It is then centrifuged at 1300 rpm for 7 minutes again, and the supernatant discarded. A 50% cell suspension is made with CRPMI washed uninfected red cells, and cultures are diluted according to parasitemia by keeping the initial parasitemia 0.5 - 1%.

The sorbitol treatment of the culture is repeated after 48 h to "fine tune" the synchronization. The synchronous culture consisting of rings, trophozoites and schizonts can be harvested at 0-18 h, 24-36 h and 36-45 h respectively after sorbitol treatment.

### Isolation of free parasites from parasitized red cells.

The infected cells from cultures are centrifuged at 3000 g for 7 minutes at 4 °C, supernatant is discarded and the packed cells are washed four times with 5-10 volumes of phosphate buffered saline, at 3000 g for 3 minutes. The washed packed cells are suspended in a known volume of PBS. To release the free parasites from infected red cells, equal volume of 0.15% saponin (in PBS) is added and the suspension is incubated at 37°C in shaking water bath with intermittent shaking for 15 min to allow complete lysis of the red cells. Ten volumes of cold PBS is added into the incubation mixture and is spun at 10,000 g for 10 min. Supernatant is discarded and the brown pellet containing the free parasites are washed extensively with PBS. The pellet of parasites can be stored at -70°C until use, as described in *Application of Genetic Engineering to Research on Tropical Disease Pathogens with Special Reference to Plasmodia; A laboratory manual of selected techniques;* S. Panyim, P. Wilairat and Yuthavong, eds., *WHO, Geneva* (1985) pp. 394.
**Apicoplast**: Apicoplast is an organelle recently described in human malarial parasite and *Toxoplasma gondii.* See, G.I. Mc Fadden et al., *Nature* **381**, 482 (1996). It is akin to the organelle plastid (found in blue green algae, plants, dinoflagellates etc.). Apicoplast in the above protozoal parasites have been speculated to have originated by the endosymbiosis of a primary endosymbiont resulting in a 3-4 layered membraneous envelope for these organelles. It has been demonstrated that these secondary endosymbionts have lost most of their genes to the host nucleus. The products of such nuclear encoded genes are targeted to apicoplast in *Plasmodium* (Consequently many of the apicoplast proteins are synthesized cytosol in the rough endoplasmic reticulum. Subsequently, they are translocated to apicoplast). Apicoplast has been implicated in the fatty acid synthetic machinery in these parasites. See, G.I. Mc Fadden et al., *Proc. Natl. Acad. Aci.* (USA) **95**, 12352-12357 (1998). Moreover, the fatty acid synthesis system in apicoplast of these parasites is reminescent of bacterial systems viz. it is of dissociative type. Indeed the Fab H gene product, β-ketoacyl-ACP synthase III, which condenses acetyl-CoA with malonyl-ACP to give acetoacyl-ACP has been identified in both, the malaria parasite (*P. falciparum*) as well as in *Toxoplasma gondii.* See, G.I. McFadden et al., *Proc. Natl. Acad Aci.* (USA) **95**, 12352-12357 (1998).
**Biosynthesis of fatty acid:** Fatty acids constitute the building blocks of phospholipids and glycolipids that make up the membranes, the defining boundaries of cells and their organelles. They also serve as stores of fuel and some of their derivatives function as inter and intracellular messengers, and precursors of vitamins and coenzymes. Intermediates, acyl groups, in fatty acid synthesis are linked covalently to an acyl carrier protein (ACP). Sequential addition of two-carbon units derived from acetyl CoA elongate the growing fatty acid chain where malonyl-ACP is the activated donor of two carbon units. The ensemble of enzyme that catalyzes the synthesis of saturated fatty acids from acetyl CoA, malonyl CoA and NADH or NADPH is called the fatty acid synthase. See, L. Stryer (1995) in *Biochemistry* W.H. Freeman and Company, San Francisco, Chapter 24.

The enzymes involved in the biosynthesis of fatty acids are organized in two distinct fashion within the living systems. See, L. Stryer (1995) in *Biochemistry* W.H. Freeman and Company, San Francisco, Chapter 24 and C.O. Rock and J.E. Cronan, *Biochim. Biophys. Acta* **1302,** 1-16 (1996). Fungi, mammals and some Mycobacteria accomplish fatty acid synthesis by multifunctional proteins in which each reaction is catalyzed by a distinct region (domain) of these very huge proteins. This class of fatty acid synthesizing enzymes are classified as type I fatty acid synthases and can also be described as the associative type of fatty acid synthases, as the successive steps in the fatty acid synthetic reaction occurs at the domains that are "hard-wired" just as the beads in a string are. Plants and many bacteria on the other hand utilize the type II or dissociated fatty acid synthase which is best characterized in the bacterium *Escherichia coli.* Each of the individual reaction in fatty acid synthesis in *E*. *coli* and plants are carried out by separate enzymes purified independently of other proteins in contrast to a single multifunctional enzyme of the mammalian system. Acetyl CoA and malonyl-ACP or acetyl-ACP and malonyl-ACP react to form acetoacetyl-ACP in a condensation reaction catalyzed by the β-ketoacyl-ACP synthase III (FabH) or acylmalonyl-ACP condensing enzyme (FabB or FabF), respectively. The condensation reaction is followed by a reduction, a dehydration and a second reduction catalyzed by β-ketoacyl-ACP reductase (FabG), β-hydroxyacetyl reductase (FabA or FabZ) and enoyl-ACP reductase (FabI), respectively, which convert acetoacetyl ACP to butyryl-ACP. Replication of this elongation cycle beginning with the condensation of the end product of the preceding cycle with malonyl ACP gives rise to the formation of C₁₆-acyl (palmitoyl)-ACP whose hydrolysis liberates palmitic acid. Palmitic acid thus formed can be either elongated by another set of enzymes or channelized for the formation of phospholipids. See, L. Stryer (1995) in *Biochemistry* W.H. Freeman and Company, San Francisco, Chapter 24.

### Detailed Description of the Preferred Embodiments

The present invention demonstrates that triclosan inhibits the growth of the human malaria parasite *Plasmodium falciparum* and the murine malaria parasite *Plasmodium berghei*.

### A. 1. Culturing of human malaria parasites

To permit detection of effect of hydroxydiphenyl ethers the inventor provides assays for culturing *Plasmodium falciparum.* The parasites can be maintained *in vitro* in human erythrocytes incubated at 37°C in CRPMI 1640 medium, which is described by W. Trager and J.B. Jensen, *Science* **173**, 673-674 (1976).

### 2. Preparation of 50% suspension of washed red cells :

Normal human erythrocytes [type O+ blood, collected in acid, citrate and dextrose (ACD)] are centrifuged for 10 min. at 1000 g. The supernatant and buffy coat are removed and the cells are washed twice with equal volume of complete RPMI (CRPMI) medium for 5 minutes at 3000 g and finally a 50 percent suspension of the washed cells is made in complete medium.

*Plasmodium falciparum* cultures with 5-6% parasitemia are diluted with washed 50% suspension of red blood cells and CRPMI so that the initial parasitemia becomes 1% and hematocrit 2%. 1.5 millilitres of this diluted infected cell suspension is placed in flat bottom vials, which are kept in a desiccator equipped with a stopcock. A candle is lit and the cover of the desiccator is put on with the stopcock open. When the candle goes out, the stopcock is closed. This is a simple but effective way to produce an atmosphere of low O₂(1-5%) and high CO₂ content (7%). Fresh medium is provided daily by withdrawing the supernatant and supplementing cells with 1.25 millilitres of CRPMI. Fresh erythrocytes are added every third or fourth day when the parasitemia reaches 6-8%, bringing it down to 1%. In cultures, all stages of the asexual cycle are seen at any one time, since the cultures have lost some of the synchronicity characteristic of *P. falciparum* in man. Delicate rings with two chromatin dots are common. Schizonts or segmenters show 15-18 merozoites, the same numbers seen in material from human infections.

### B. Morphological examination of parasites.

This is done by observing the stained smear of infected red blood cells under the microscope in oil immersion. The following stages of *Plasmodium falciparum* with their characteristic features can be seen. These stages are as follows:
1. **Rings:** This is characterized by its signet ring appearance. The nucleus is stained red, whereas the cytoplasm takes up blue and appears as a thin circle.
2. **Trophozoites:** Most of the host red blood cells is occupied by the dense cytoplasm of parasite at this stage. Trophozoites have characteristic brownish yellow malaria pigment and a food vacuole. Late trophozoites have 3-4 nuclei and undivided cytoplasm.
3. **Schizonts:** Most of the red blood cell is occupied by 12-13 nuclei present at this stage of the parasite. Each of the nucleus is surrounded by cytoplasm. The smears also contain host red blood cells (RBC) which, do not take up stain and are without nucleus.

*P. berghei* smears have in addition reticulocytes. *P. berghei* preferentially invade reticulocytes. Different types of white blood cells such as lymphocytes, neutrophils etc. also appear in smear of *P*. *berghei.* They are generally larger than erythrocytes or reticulocytes and take up deep blue stain.

### C. Determination of IC₅₀ for Inhibition of parasite growth.

Demonstration of triclosan as an antimalarial *in vitro* is one of the aspect of present invention. This necessitates assessing the ability of this compound in killing the parasite *in vitro.* The IC₅₀ for a drug can be assayed in a 96 well titre plate. See, R.E. Desjardins et al., *Antimicrob. Agents Chemother.* **16**, 710-718 (1979). Triclosan is dissolved in a suitable solvent at an appropriate concentration. 200 µl aliquots of infected red blood cells with 4-7% parasitemia and 1.5 - 2% hematocrit is aliquoted in wells according to requirement. 25 µl of drug with highest concentration to be added is pipetted in the first well. After making serial double dilutions across the plate, 25 µl of the diluted drug solution is added to rest of the required number of wells. Each concentration was tested in triplicate wells. The plates were placed in a desiccator and incubated for 24 h at 37°C. Fresh medium and drug was added every 24 h, if the effect of the drug was to be studied after 48 or 72 h.

### D. Preparation of isotope and labeling of parasites.

Uptake of [³⁵S] methionine, NEN, DuPont, (specific activity 1175 Ci/mmol) is used as an index of parasite growth. The final isotope concentration to be added in each well is 100 µCi/ml culture. Before an hour of total incubation period (24, 48, or 72 h) 20 µl of the isotope in culture medium is added to each well and incubated for an additional 1 hr. After incubation, contents of the well are pipetted into microcentrifuge tubes kept on ice. The samples are spun at 3000 g for 10 sec. The supernatant is discarded and the pellet washed twice in 1.0 ml of 0.9% NaCl. The pellet is lysed with 50 µl distilled water and spotted onto Whatman No. 3 discs (presoaked in 10% TCA with cold methionine and dried). After spotting, these discs are allowed to dry and then they are treated with hot TCA, cold TCA (10% throughout), alcohol ether (2:1, v/v) and finally with ether. During hot TCA treatment, hydrogen peroxide solution (30%, v/v) is added to a final concentration of 3% for bleaching purpose. Duration of each treatment is 7 minutes. The discs are air dried after ether treatment, and counted in optiphase 'Hisafe' scintillation fluid (Wallac Scintillation Products, U.K.) in a Wallac β-counter (Model 1409, Finland).

### E. Effect of triclosan in vivo:

The present invention involves the use of triclosan for the manufacture of an antimalarial agent to inhibit the growth of the murine malaria parasite *Plasmodium berghei* as well. The effect of various concentration of triclosan in controlling parasitemia is monitored in *Plasmodium berghei* infected mice.
***1. Plasmodium berghei* infection:** *P. berghei* parasite is maintained in Swiss male mice through serial blood passage.
**2. Triclosan treatment:** Swiss mice (5 animals/group) are infected on day zero with about 10⁶ infected red blood cells (IRBC). The animals are kept under observation to record parasitemia and mortality. Parasitemia is determined by counting 10³ red cells in a thin blood smear stained with Giesma and expressed as number of parasitized cells/100 erythrocytes. The animals given only dimethylsulfoxide (DMSO) are used as controls and referred to as untreated animals. Level of parasitemia is >1% on the first day of triclosan treatment. Parasitemia is monitored every 24 hrs, for 7-8 days. The untreated animals die by 8 days after the infection, whereas delayed mortality is observed in treated animals depending on dose.

### F. Triclosan as antimalarial agent.

The invention identifies triclosan (See the structure shown below) and other hydroxydiphenyl ethers, the widely used anti-bacterial agents as lead compounds for developing powerful anti-malarial agents as well as fatty acid biosynthesis pathway as a target for anti-malarial chemotherapy.

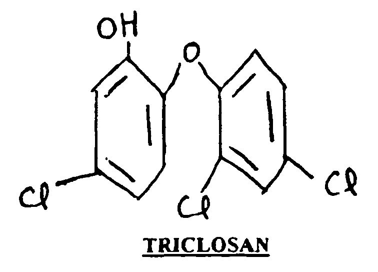

Additionally, FabI is identified as the enzyme in malaria, targeted by triclosan, a hydroxydiphenyl ether. Indirect evidences strongly point to the presence of dissociative type fatty acid synthase in the malaria parasite. See, G.I. McFadden et al., *Proc. Natl. Acad. Aci.* (USA) **95**, 12352-12357 (1998). Hence the elongation cycle of fatty acid synthesis in malaria parasite in analogy to the bacterial system can be proposed to consist of the reactions shown schematically below.

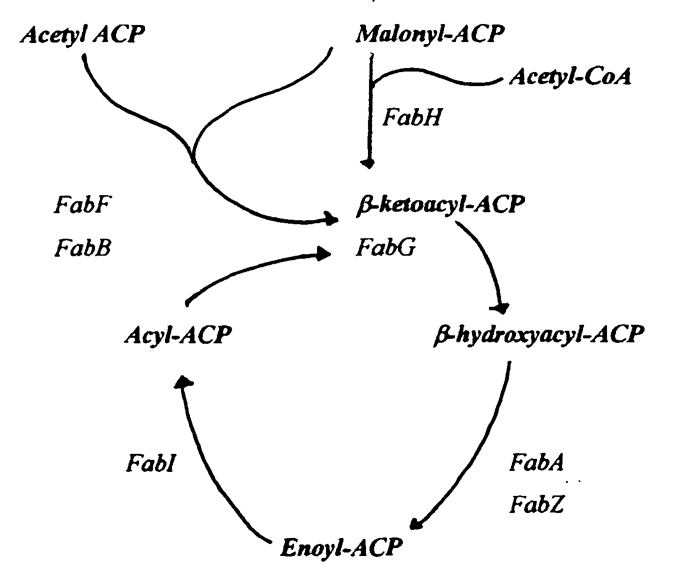

These are namely the condensing reaction catalyzed by β-ketoacyl-ACP synthase III (FabH), reduction of β-ketoacyl-ACP by a reductase (FabG) to yield β-hydroxyacyl-ACP, which is dehydrated by β-ketoacyl dehydrase (FabZ), enoyl-ACP thus formed is reduced by enoyl-ACP reductaste (FabI). Thus at the end of each elongation cycle of acyl-ACP with two methylene groups i.e. -(CH₂)₂- are added. The elongation cycle terminates when palmitoyl CH₃-(CH₂)₁₄-C-S-ACP is formed. Palmitic acid CH₃-(CH₂)₁₄-COOH, is then liberated from palmitoyl-ACP by a hydrolase. Of the four reactions of the elongation cycle of fatty acid synthase, existence of the genes for two of them, FabH and FabZ, have been demonstrated recently in *P. falciparum.*

However, as several gene products could be involved in the condensation and dehydration reactions the gene products of FabH and FabZ though important for fatty acid synthesis are perhaps not very indispensable. See, C.O. Rock and J.E. Cronan, *Biochim. Biophys. Acta* **1302,** 1-16 (1996).

It has been demonstrated convincingly in bacterial systems that only one enzyme is involved in the reduction of enoyl-ACP i.e. the FabI gene product, enoyl-ACP reductase. More so, it has been established that enoyl-ACP reductase is the rate determining factor in completing rounds of fatty acid elongation. See, R.J. Heath and C.O. Rock, *J. Biol. Chem.* **270,** 26538-26542 (1995) and R.J. Heath and C.O. Rock, *J. Biol. Chem.* **271,** 1833-1836 (1996). Hence it constitutes a key regulatory element in any dissociated fatty acid synthase system. Moreover, it has also been shown that Triclosan targets FabI gene product in *E. coli.* See, L.M. McMurray et al. *Nature* **394,** 531-532 (1998) and R.J. Heath et al. *J. Biol. Chem.* **273,** 30316-30320 (1998). As the fatty acid synthesis system of malaria parasite is of dissociated type, it is surmized by us that inhibitors of enoyl-ACP reductase would prove potent anti-malarial agents.

2-Hydroxydiphenyl ethers are a class of compounds which display wide spectrum anti-bacterial activity. A number of these compounds have been used in a wide variety of consumer products including treatment of textiles etc. Of these compounds triclosan is the most effective microbiocide and is used commonly in toothpastes, soaps, deodorants, oral rinses, ointment for skin and other dermatological formulations and also to some extent in plastics for children's toys. See, H.N. Bhargava and P.A Leonard *Am. J. Infect. Control.* **24**, 209-218 (1996).

However, the present invention very clearly shows that triclosan exhibits a very potent antimalarial activity.

### G. Inhibition of fatty acid synthesis in the malaria parasite by Triclosan

In the previous section it has been demonstrated that triclosan inhibits the growth of the human malaria parasite *in vitro* in a very potent manner. To elucidate further its mode of action, its effects on the incorporation [¹⁴C]-malonyl-CoA a key intermediate required for fatty acid synthesis, in the fatty acids in a cell free system of malaria parasite is demonstrated. For cell free fatty acid synthesis in *E.Coli,* See Δ P.W. Majerus et al. (1968), *Methods in Enzymol,* **14**, 43-52.

These experiments clearly show that Triclosan inhibits incorporation of [¹⁴C] malonyl CoA in fatty acids in cell free system for fatty acid synthesis in this parasite. A progressive decline in the longer chain fatty acids in the presence of triclosan clearly implicates FabI of the malaria parasite as the target, as it plays a deterministic role in the elongation cycle of the fatty acid synthesis. This invention thus, also embraces the use of hydroxydiphenyl ethers such as triclosan or the other compounds of this class that may be developed in future as inhibitors of this enzyme thereby acting as anti-malarial agents as well as any other class of molecules that impinge on this enzyme activity.

### EXAMPLES

### Example 1: In vitro Effect of Triclosan on growth of Plasmodium falciparum.

200 µl *P. falciparum* cultures with 10% parasitemia at specific stage (viz. ring or trophozoites obtained after sorbirol treatment) were aliquoted in required number of wells in a 96 well microtitreplate. Stock solution of triclosan was made in DMSO. Further dilutions of triclosan were made in a solvent mixture comprising of DMSO : Methanol : Water in 1 : 1 : 3 (v/v) ratio. The drug was further diluted in complete RPMI, so that the final concentration of DMSO is 0.005%. This concentration of DMSO has no inhibitory effect by itself on growth of parasites. Drug in 10 µl (0.5 µg-0.0019 µg / 200 µl cultures) was added in each well by serial double dilutions. The plates were placed in the desiccator and incubated at 37°C according to J.B. Jensen and W. Trager, *Science* **173**, 673-675 (1976). Each drug concentrations was tested in triplicate wells. Control wells with 0.005% DMSO as solvent as well as no solvent, were also taken as untreated cultures. Medium was changed every 24 h and fresh medium along with the drug were added. Giemsa stained smears were examined for the parasitemia as well as morphology of parasites.

### Example 2: Measurement of uptake of (³⁵S) methionine, as an index of inhibition of parasite growth.

The drug and parasites were aliquoted in required number of rows of wells in 96 well titre plates as described in example 1. [³⁵S] Methionine (20 µCi/well) was added in each well for 1 hour before required time point of harvesting (24, 48 or 72 h). Content from each well was transferred to microcentrifuged tubes on ice and spun at 3000 g for 10 seconds. The supernatant was discarded and the infected red blood cell (IRBC) pellet was washed four times with 0.5 ml 0.9% NaCl. The washed pellet was lysed with 50 µl distilled water and spotted onto Whatman No. 3 discs (presoaked in 10% TCA with cold methionine and dried). After drying, these discs were treated with hot TCA, hot TCA with 3% hydrogen peroxide for bleaching, cold TCA, alcohol : ether (2 : 1, v/v) and finally ether, (in that sequential order) each for 7 minutes. After ether treatment, the discs were air dried, placed in scintillation fluid phase III from Wallac and counted in Wallac β-counter.

### Example 3: Protection of mice against malaria after administration of Triclosan in vivo.

Male swiss mice were infected with *P. berghei.* The animals were kept under observation and parasitemia was recorded daily. Triclosan (0.8, 1.6, 3.0, 8.0, 14.0 and 28.0 mg/kg body weight of mice respectively) in DMSO was given subcutaneously on day 1 of infection when parasitemia was>1% and subsequently for the next 6 days. Experiments with triclosan were conducted with a group of five animals, each for the dosages mentioned above. DMSO was given to control animals (6) and were referred to as untreated animals. Parasitemia and mortality were recorded till the untreated mice died. Typically all the six mice in the control group died by day 9 of infection while 3, 3 and 4 mice out of 5 treated with 0.8, 1.6 and 3.0 mg triclosan/kg (body weight), survived till day 14. All the mice (5 out of 5) survived till 14^{th} day when treated with 8.0, 14.0 and 28.0 mg triclosan/kg (body weight).

### Example 4: Incorporation of [¹⁴C] malonyl-CoA in fatty acids and its inhibition by Triclosan in vitro.

Synchronized cultures of *P. falciparum* (100 ml) exhibiting 10-12% parasitemia, at the late ring stage were harvested by centrifugation at 3000 g for 5 min. The pellet was washed four to five times with cold phosphate buffer saline and after washing, the pellet was suspended in 3.0 ml of phosphate buffer saline and equal volume of 0.15% saponin was added to isolate free parasites. (See specific embodiments). The late ring stage (trophozoites) of the parasites thus isolated were washed once thoroughly with PBS and the cells suspended in 0.4 ml of 1 mM potassium phosphate buffer pH 7.0 and sonicated for 15 sec. The cell lysate (2.0 mg cell extract protein) thus prepared was mixed with 200 µl of 200 mM of potassium phosphate buffer pH 7.0 containing 70 uM acetyl CoA, 8 mM glucose-6-phosphate, 3 mM each of NADH and NADPH, 0.4 mM EDTA, 4 units of glucose-6-phosphate dehydrogenase, 45 uM [¹⁴C]malonyl-CoA (specific activity 54.2 mCi/mmol, DuPont) and 3 mM dithiothreitol (DTT). To this 200 µg of DTT reduced acyl carrier protein (ACP) from *E*. *coli* (Sigma) in 25 µl of 75 mM phosphate buffer was added. The reaction mixture was incubated for 30 min at 37°C. Reactions were also conducted under identical conditions with 0.15, 0.225, 0.3, 0.6 and 1.2 µg of triclosan which was added to the reaction mixture just prior to the addition of reduced ACP solution. To the reaction mixture 5 ml of 4 M HCl was added and the samples incubated at 100°C for 2 h. The fatty acids thus liberated were extracted in chloroform. After evaporation of the solvent the residue was dissolved in ether and methylated at 4°C with diazomethane in ether. Ether was evaporated then and the methyl esters dissolved in 75 ul of methanol of which 25 ul of the samples were spotted on a sialinized silica thin-layer plates (E.Merck AG, Germany) and the mixture chromatographed with acetone-methanol-water-acetic acid (70:50:35:1, vol/vol/vol/vol). The air dried plates were visualized with autoradiography. A part (25 µl) of the sample in methanol was counted by scintillation counting to determine also the extent of incorporation of [¹⁴C] malonyl-CoA in fatty acids both in the presence and absence of Triclosan.

These experiments reveal that Triclosan not only inhibits the incorporation of [¹⁴C] malonyl CoA in fatty acids but also the elongation reaction of fatty acid synthesis is targeted in a striking manner.

## Claims

1. Use of Triclosan (2, 4, 4- trichloro-2-hydroxydiphenyl ether) or a pharmaceutically acceptable derivative thereof for the manufacture of a medicament to inhibit the growth of the malaria parasite human *Plasmodium falciparum* or murine *Plasmodium berghei.*

2. Use as claimed in Claim 1 in targeting the Fab I enzyme, enoyl-ACP reductase, in the malaria parasite.

3. Use as claimed in any of Claims 1 - 2 in inhibiting the elongation reaction of fatty acid synthesis in malaria parasite.

4. Use as claimed in any of Claims I to 3 wherein the medicament is suited for treatment by introduction in the blood by any method or for application by any injectable route, preferably intramuscular or intradermal or intraperitoneal or intravenous or intro-arterial or subcutaneous.

5. Use as claimed in any of Claims 1 to 4 wherein the medicament comprises an injectable composition consisting of Triclosan (2, 4, 4-trichloro-2-hydroxydiphenyl ether) or a pharmaceutically acceptable derivative thereof and a pharmaceutically acceptable adjuvant, or a diluent or a carrier, the composition being suitable for introduction in the blood by any method or for application by any injectable route, preferably intramuscular or intradermal or intraperitoneal or intravenous or intro-arterial or subcutaneous route.

6. A method of testing to confirm that the growth of human malaria parasite Plasmodium falciparum is inhibited by the use of Triclosan (2, 4, 4- trichloro-2-hydroxydiphenyl ether), comprising
a. Examining smears of *in vitro* treated cultures for morphological features of the parasite as an indicator of growth, or
b. Monitoring the incorporation of [³⁵S] methionine in infected red blood in vitro as a quantitative indicator of the inhibition of the parasite growth.

7. A method of determining if the growth of animal malaria parasite murine *Plasmodium berghei* is inhibited by the injectable composition as claimed in Claim 5, said method comprising:
a Monitoring the extent of inhibition of parasitemia by examining the smears of blood taken from an animal.

## Patentansprüche

1. Die Verwendung von Triclosan (2,4,4-Trichlor-2-hydroxydiphenylether) oder eines pharmazeutisch akzeptablen Derivats davon zur Herstellung eines Medikaments zur Hemmung des Wachstums des Malariaparasiten Human-*Plasmodium falciparum* oder Maus-*Plasmodium berghei.*

2. Die Verwendung gemäß Anspruch 1, abzielend auf das Fab I Enzym, Enoyl-ACP Reduktase, in dem Malariaparasiten.

3. Die Verwendung gemäß einem der Ansprüche 1-2 zur Hemmung der Verlängerungsreaktion bei der Fettsäuresynthese in dem Malariaparasiten.

4. Die Verwendung gemäß einem der Ansprüche 1 bis 3, wobei das Medikament zur Behandlung durch Einbringen in das Blut mittels jeglicher Methode geeignet ist, oder zur Applikation über jeglichen Injektionsweg, vorzugsweise intramuskulär oder intradermal oder intraperitoneal oder intravenös oder intra-arteriell oder subcutan.

5. Die Verwendung gemäß einem der Ansprüche 1 bis 4, wobei das Medikament eine injizierbare Zusammensetzung enthält bestehend aus Triclosan (2,4,4-Trichlor-2-hydroxydiphenylether) oder einem pharmazeutisch akzeptablen Derivat davon, und einem pharmazeutisch akzeptablen Hilfsstoff, oder einem Verdünnungsmittel oder einem Trägerstoff, wobei die Zusammensetzung zum Einbringen in das Blut mittels jeglicher Methode oder zur Applikation über jeglichen Injektionsweg geeignet ist, bevorzugt intramuskulär oder intradermal oder intraperitoneal oder intravenös oder intra-arteriell oder subcutan.

6. Ein Testverfahren um zu bestätigen, dass das Wachstum des Human-Malariaparasiten *Plasmodium falciparum* durch die Verwendung von Triclosan (2,4,4-Trichlor-2-hydroxydiphenylether) gehemmt wird, umfassend
a. Untersuchen der Abstriche von *in vitro* behandelten Kulturen auf morphologische Besonderheiten der Parasiten als Indikator für Wachstum, oder
b. Überwachen des Einbaus von [³⁵S] Methionin in infiziertes rotes Blut in vitro, als quantitativer Indikator der Hemmung des Parasiten-Wachstums.

7. Ein Verfahren zur Bestimmung, ob das Wachstum von tierischem Malariaparasiten Maus-*Plasmodium berghei* gehemmt wird durch die injizierbare Zusammensetzung wie in Anspruch 5 beansprucht, umfassend:
a. Überwachen des Ausmasses der Hemmung von Parasitämie durch Untersuchen der Blutabstriche, die von einem Tier genommen wurden.

## Revendications

1. Utilisation du triclosan (éther 2,4,4-trichloro-2-hydroxydiphénylique) ou d'un dérivé pharmaceutiquement acceptable de celui-ci pour la fabrication d'un médicament destiné à inhiber la prolifération du parasite paludéen *Plasmodium falciparum* humain ou *Plasmodium berghei* murin.

2. Utilisation selon la revendication 1 pour cibler l'enzyme Fab I, énoyl-ACP réductase, dans le parasite paludéen.

3. Utilisation selon l'une quelconque des revendications 1-2 pour inhiber la réaction d'allongement de la synthèse d'acides gras chez le parasite paludéen.

4. Utilisation selon l'une quelconque des revendications 1 à 3, dans laquelle le médicament est utilisable pour le traitement par introduction dans le sang par un procédé quelconque ou par application par toute voie injectable, de préférence, intramusculaire ou intradermique ou intrapéritonéale ou intraveineuse ou intra-artérielle ou sous-cutanée.

5. Utilisation selon l'une quelconque des revendications 1 à 4, dans laquelle le médicament comprend une composition injectable constituée de triclosan (éther 2,4,4-trichloro-2-hydroxydiphénylique) ou d'un dérivé pharmaceutiquement acceptable de celui-ci et d'un adjuvant, ou d'un diluant ou véhicule pharmaceutiquement acceptable, la composition pouvant être introduite dans le sang par un procédé quelconque ou appliquée par toute voie injectable, de préférence, intramusculaire ou intradermique ou intrapéritonéale ou intraveineuse ou intra-artérielle ou sous-cutanée.

6. Méthode d'essai pour confirmer que la prolifération du parasite paludéen humain *Plasmodium falciparum* est inhibée par l'utilisation du triclosan (éther 2,4,4-trichloro-2-hydroxydiphénylique), comprenant les étapes consistant à :
a. examiner des frottis de cultures traitées *in vitro* pour détecter des traits morphologiques du parasite à titre d'indicateur de prolifération, ou
b. surveiller l'incorporation de [³⁵S] méthionine dans des érythrocytes infectés *in vitro* à titre d'indicateur quantitatif de l'inhibition de la prolifération du parasite.

7. Procédé pour déterminer si la prolifération du parasité paludéen animal *Plasmodium berghei* murin est inhibée par la composition injectable selon la revendication 5, ledit procédé comprenant les étapes consistant à :
a. Surveiller le degré d'inhibition de la parasitémie en examinant les frottis de sang prélevé sur un animal.
